**Europäisches Patentamt**

**European Patent Office**

**Office européen des brevets**

(19)

(11) Publication number: **0 087 171**
**A1**

(12) # EUROPEAN PATENT APPLICATION

(21) Application number: **83103270.1**

(22) Date of filing: **08.05.81**

(51) Int. Cl.³: **A 61 M 1/03**

(30) Priority: **27.06.80 SE 8004795**

(43) Date of publication of application:
**31.08.83 Bulletin 83/35**

(84) Designated Contracting States:
**AT BE CH DE FR GB IT LI LU NL SE**

(60) Publication number of the earlier application
in accordance with Art. 76 EPC: **0 042 939**

(71) Applicant: **Gambro Lundia AB**
**Box 10101**
**S-220 10 Lund(SE)**

(72) Inventor: **Shaldon, Stanley**
**Villa 86 Rue de Grézac**
**F-34100 Montpellier(FR)**

(72) Inventor: **Gullberg, Claes-Ake**
**380 E Dexter**
**Covina, CA 91723(US)**

(72) Inventor: **Larsson, Lars-Ake**
**Odlarevägen 66**
**S-241 00 Löddeköpinge(SE)**

(74) Representative: **Boberg, Nils Gunnar Erik et al,**
**Gambro AB Patent Department Box 10101**
**S-220 10 Lund(SE)**

(54) Safety system for control of a filter.

(57) Safety system for controlling a filter (10) with an inlet (11) and an outlet (12) for the liquid which is to be filtered and an outlet (13) for the filtrate. The filter (10) is arranged between a liquid treating system (1-9) and a sterile system (18-23) adapted to receive the treated and filtered liquid. A recirculation circuit (14) is arranged in parallel with the filter (10) and connected to the first mentioned inlet (11) and outlet (12) and has means (16, 17) for the supply of an indicator which is not normally allowed passage through the filter (10). The system further includes a detector (19) for the filtrate, disposed after the outlet (13) for the filtrate, said detector sensing if the indicator has nevertheless been allowed to pass together with the filtrate into the sterile system (18-23).

Preferably the safety system is intended to be used in connection with a hemofiltration system.

Fig.1

EP 0 087 171 A1

## TITLE OF THE INVENTION
### SAFETY SYSTEM FOR CONTROL OF A FILTER

## TECHNICAL FIELD

The present invention relates to a safety system for controlling a filter with an inlet and an outlet for the liquid which is to be filtered and an outlet for the filtrate, the filter being arranged between a liquid treating system and a sterile system adapted to receive the treated and filtered liquid.

Preferably the safety system according to the present invention is intended to be used in connection with the hemofiltration system according to European patent application 81.103504.7 published under No.EP 00 42 939, but for the man skilled in the art it would be clear that it can also be used in connection with many other situations when it is desirable to be absolutely sure about the function of a filter arranged between a sterile system and a system which is not guaranteed to be sterile.

## THE STATE OF THE ART

In connection with for instance hemodialysis it is well known to arrange after the dialyser different kind of photoelectric cell devices in order to detect possible blood leakages through the filter. The risk for a leakage in the opposite direction is very small due to the fact that a negative pressure is used at the dialysis liquid side of the membranes.

Consequently, possible leakages occur from the sterile side to the non sterile side and not in the opposite direction.

Furthermore, the US patent 4 081 372 describes a system for peritoneal dialysis in which the dialysis liquid used is purified by means of dialysis. Due to the fact that the peritoneal dialysis is carried out without direct contact with the blood hemoglobin is in this case added as an artificial indicator. In connection with a possible rupture of the dialysis membrane such a rupture is detected by means of a photoelectric cell device. Also this system functions satisfactor connection with a positive pressure in the sterile part. A possible leakage from the non sterile part to the sterile part is not detected.

## DESCRIPTION OF THE PRESENT INVENTION

The present invention is instead intended to provide a system in which a liquid can be fed in a safe way and in a sterile condition from a liquid treating system via a filter to a sterile system having a lower pressure.

This is made according to the invention by means of a safety system of the above defined kind characterized by a recirculation circuit arranged in parallel with the filter and connected to the first-mentioned inlet and outlet and having means for the supply of an indicator which is not normally allowed passage through the filter, and by a detector for the filtrate, disposed after the outlet for the filtrate, said detector sensing if the indicator has nevertheless been allowed to pass together with the filtrate into the sterile system.

The recirculation circuit may here contain a recirculation pump for ensuring that the indicator is constantly caused to pass the membrane disposed in the filter.

The indicator may, in a simple manner, be supplied to the recirculation circuit if this contains a wall portion which allows the passage of an injection syringe or the like.

As indicator, use may be made of a colourant, for example blue dextrane. The safety system is here provided with photocell sensing device disposed, in the direction of flow, after the outlet for the filtrate.

## BRIEF DESCRIPTION OF THE ACCOMPANYING DRAWINGS

The present invention will be described in greater detail below with reference to the accompanying drawings which illustrate three different hemofiltration systems according to the invention.

Fig. 1 shows a first hemofiltration system with continuous preparation of replacement liquid.

Fig. 2 shows a second hemofiltration system according to the invention with batchwise preparation of the replacement liquid. When a portion has been prepared, this is continuously supplied to the patient.

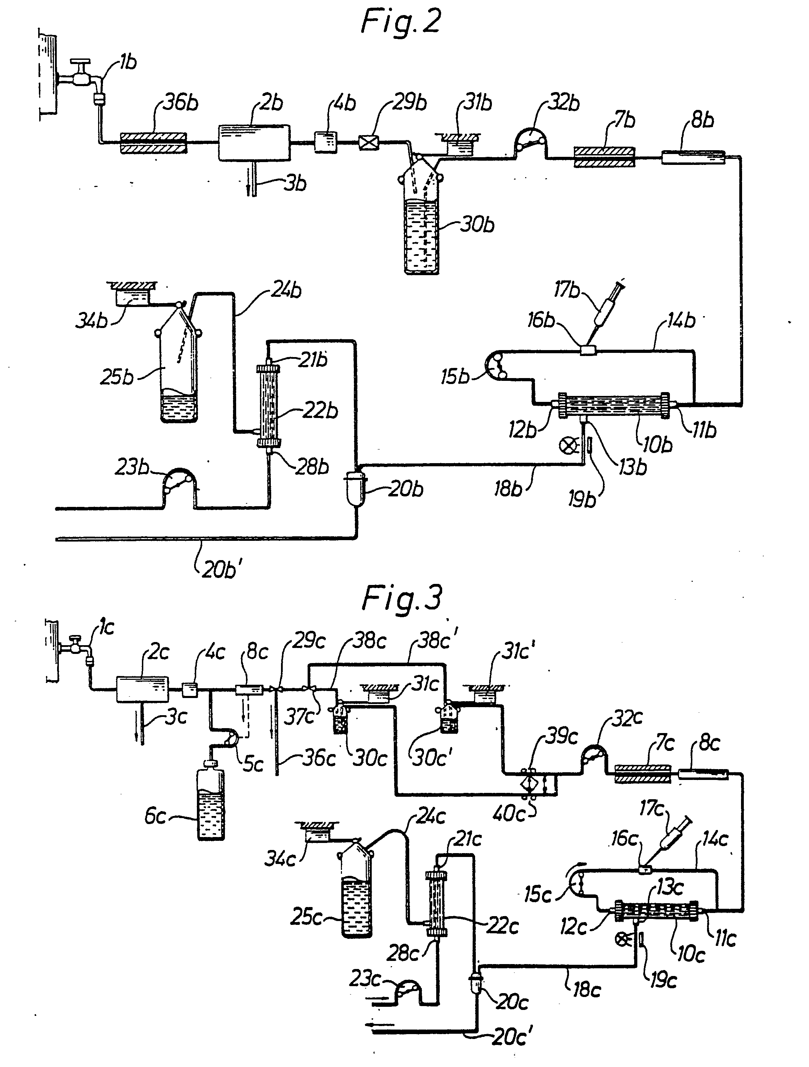

Finally, Fig. 3 shows a third hemofiltration system with 0087171 continuous preparation of replacement liquid which may be divided into portions which are then continuously supplied to the patient.

## HEMOFILTRATION WITH CONTINUOUS PREPARATION OF REPLACEMENT LIQUID

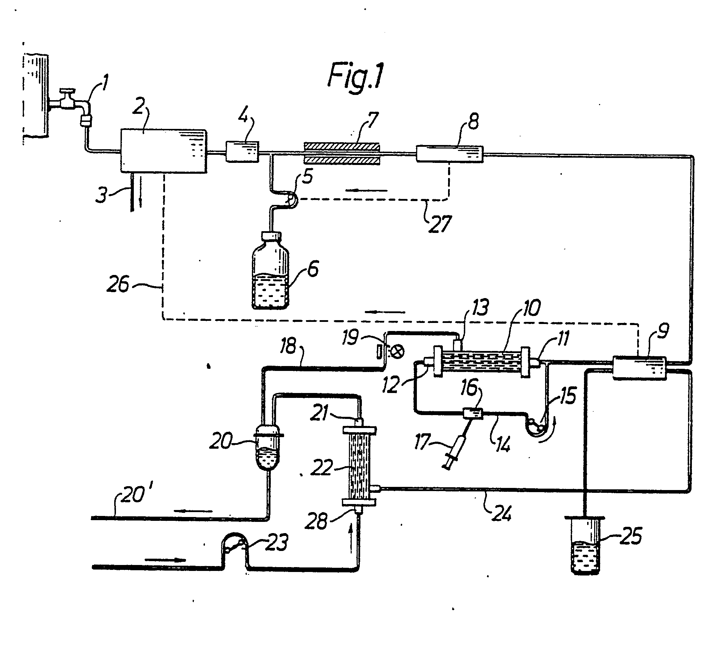

In Fig. 1, a water conduit connection is designated 1, for example a normal water faucet. The water is led thence into a module 2 for reverse osmosis, whence waste water is drained off via a conduit 3. The purified water is instead passed through a container 4 which is filled with activated charcoal and is intended for adsorption of chlorine and pyrogenes. Concentrate for the replacement liquid is dosed by means of a pump 5 from a container 6 in order to realize an isotonic solution. This solution is led through a heating device 7 and a conductivity metre 8 which in its turn controls the pump 5.

The prepared solution is then led through a flowmetre 9 to a filter 10 with an inlet 11 and an outlet 12 for the liquid which is to be filtered, as well as an outlet 13 for the filtrate. The filter 10 may, for example, consist of a normal hemofilter containing fibres of a semipermeable material.

A recirculation circuit 14 is connected to the inlets and outlets 11 and 12 and has a pump 15 and an injection site 16. At the injection site 16, an indicator, for example blue dextrane may be injected by means of a normal injection syringe 17. The outlet 13 is connected to a conduit 18 which includes a photocell sensing device 19. Should a leak occur in the filter 10, this would, thus be discovered directly such that measures may be taken.

The conduit 18 discharges into a drip chamber 20 to which the outlet side 21 of a hemofilter 22 is also connected. From the drip chamber 20, the mixture of replacement liquid and blood is led via the conduit 20' to the patient.

The hemofilter 22 is fed by means of a pump 23 with blood from the patient (not shown). Plasma or ultrafiltrate is removed in a conventional manner through a conduit 24 by the intermediary of the flowmetre 9 to a receptacle site 25. The broken line 26 intimates how the flowmetre 9 may be disposed to control the module 2 for reverse osmosis. In the sammer manner, the line 27 intimates that the conductivity metre 8 may be disposed to control the pump 5 for pumping concentrate from the container 6 to the main conduit. Finally, the blood inlet for the hemofilter 22 is designated 28.

## HEMOFILTRATION WITH BATCHWISE PREPARATION OF REPLACEMENT LIQUID

The system according to Fig. 2 corresponds in essential parts to the systems according to Fig. 1. Hence, the same reference numerals have been used here, but with the addition of a _b_. Thus, the following details are to be found in the system:

1b  - connection point for water conduit water, for example a normal water faucet

36b - preheater

2b  - module for reverse osmosis

3b  - outlet for impure water

4b  - cartridge with activated charcoal

29b - shut-off valve

30b - container for replacement liquid

31b - scales for replacement liquid

32b - pump for replacement liquid

7b  - heating device

8b  - conductivity metre

10b - filter

11b - inlet for filter

12b - outlet for filter

13b - outlet for filtrate

14b - recirculation circuit

15b - recirculation pump

16b - injection site for indicator

17b - injection syringe for indicator

18b - conduit

19b - photocell sensing device

20b - drip chamber

20b'- return conduit to patient

21b - blood outlet for hemofilter

22b - hemofilter

28b - blood inlet for hemofilter

23b - pump for blood

24b - outlet conduit for the hemofiltrate

25b - receptacle container for the hemofiltrate

33b - scales for the hemofiltrate

The system according to Fig. 2 differs from the system according to Fig. 1 primarily in that the replacement liquid is prepared in portions i the container 30b. By the preparation the concentrate may either be supplied to the container before the start of the preparation of during the preparation. If necessary, the container may be completed with suitable devices for stirring the prepared liquid. When a suitable amount of the liquid is provided in the container 30b the valve 29b is closed. The hemofiltration proper can thereafter be started. The supply to the patient is made in essentially the same way as by the system according to Fig. 1, i.e. through a filter 10b provided with a safety system according to the present invention and then via a drop chamber 20b. At the same time the hemofiltrate is fed via the outlet conduit 24b to the container 25b for weighing by means of the weigh 34b.

HEMOFILTRATION WITH CONTINUOUS BATCHWISE PREPARATION OF REPLACEMENT LIQUID AND CONTINUOUS SUPPLY TO THE PATIENT

The system according to Fig. 3 also corresponds in essential details to the systems according to Figs. 1 and 2. The same reference numerals have, therefore, also been used here, but with addition to the letter c. Thus, the following details are present in this system:

1c - connection point for water conduit water, for example a normal water faucet

2c - module for reverse osmosis

3c - outlet for impure water

4c - cartridge with activated charcoal

5c - pump

6c - container for concentrate

8c - conductivity metre

29c - shut-off valve

36c - discharge conduit for liquid of incorrect composition

37c - valve

38c - conduit to a first dosage container

30c - first dosage container

0087171

38c'- conduit to second dosage container

30c'- second dosage container

31c - scales for first dosage container

31c'- scales for second dosage container

39c - valve

40c - valve

32c - pump for replacement liquid

7c  - heating device

8c  - conductivity metre

10c - filter

11c - inlet

12c - outlet

13c - outlet for the filtrate

14c - recirculation circuit

15c - recirculation pump

16c - injection site for indicator

17c - injection syringe for indicator

18c - conduit

19c - photocell sensing device

20c - drip chamber

20c'- return conduit to the patient

21c - blood outlet for hemofilter

22c - hemofilter

28c - blood inlet for hemofilter

23c - blood pump

24c - outlet conduit for the hemofiltrate

34c - scles for the hemofiltrate

The system according to Fig. 3 may be described as a combination of earlier described systems. Replacement liquid is here continuously prepared by means of details 1c, 2c, 3c, 4c, 5c, 6c and 8c. Should the conductivity be incorrect, the liquid is removed via the conduit 36c, thanks to the switch valve 29c. On the other hand, if the conductivity metre 8c shows that the right composition has been obtained, the liquid is passed via the valve 37c and the conduit 38c to a first dosage container 30c which is weighed by means of scales 31c. When a suitable amount of liquid has arrived at this container, it is fed further, at the same time as newly-prepared liquid instead is passed via the valve 37c and the conduit 38c' to a second dosage container 30c' which weighed by means of scales 31c'. Here, the valve 39c is closed, whereas the previously prepared is passed via the valve 40c and the pump 32c through the heating device 7c and the conductivity metre 8c, via the filter 10c and the drip chamber 20c to the patient. The filter 10c is, here, provided with a safety system of the above-described type. When the container 30c has been emptied, new liquid has been prepared in the container 30c'. The valves 37c, 39c and 40c may here be switched for discharge of the newly-prepared liquid. During the entire time, blood is continuously pumped by means of the pump 23c through the hemofilter 22c and via the drip chamber 20c back to the patient. At the same time, hemofiltrate is removed via the conduit 24c and is collected in the container 25c which is weighed by means of scales 34c.

Naturally, the present invention is not restricted merely to the above-described examples, but may be varied within the spirit and scope of the appended claims.

The safety system according to the invention may for instance as above mentioned also be used for other purposes such as for preparing of other infusion solutions.

8

0087171

CLAIMS

1. Safety system for controlling a filter (10) with an inlet (11) and an outlet (12) for that liquid which is to be filtered and an outlet (13) for the filtrate, the filter (10) being arranged between a liquid treating system (1-9) and a sterile system (18-23) adapted to receive the treated and filtered liquid, c h a r a c t e r i z e d by a recirculation circuit (14) arranged in parallel with the filter (10) and connected to the first-mentioned inlet (11) and outlet (12) and having means (16, 17) for the supply of an indicator which is not normally allowed passage through the filter (10), and by a detector (19) for the filtrate, disposed after the outlet (13) for the filtrate, said detector sensing if the indicator has nevertheless been allowed to pass together with the filtrate into the sterile system (18-23).

2. Safety system according to claim 13, c h a r a c - t e r i z e d in that the recirculation circuit contains a recirculation pump (15).

3. Safety system according to claim 14, c h a r a c - t e r i z e d in that the recirculation circuit further includes a wall portion (16) which allows the passage of an injection syringe (17).

4. Safety system according to any one of the preceding claims with a colourant as indicator, for exampe blue dextrane, c h a r a c t e r i z e d by a photocell sensing device (19) for the filtrate, disposed in the flow direction after the outlet (13) for the filtrate.

5. Use of a safety system according to any one of the preceding claims, c h a r a c t e r i z e d in that it is used for control of the sterility of an infusate solution in a hemofiltration system before this solution leaving the system in order to be supplied to the patient, the infusate solution being caused to pass through the filter (10) in order to be injected into the patient downstream of the detector (19).

Fig.1

## Fig.2

## Fig.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,D | US-A-4 081 372 (C. LYMAN ATKIN et al.) <br> * Figures; column 2, line 58 - column 3, line 8 * | 1,2,4 | A 61 M 1/03 |
|  | ----- | |  |

TECHNICAL FIELDS
SEARCHED (Int. Cl. 3)

A 61 M

The present search report has been drawn up for all claims

| Place of search THE HAGUE | Date of completion of the search 15-06-1983 | Examiner VEREECKE A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82